# EUROPEAN PATENT APPLICATION

(11) **EP 4 406 941 A1**
(43) Date of publication of application: **31.07.2024**
(21) Application number: 24153555.8
(22) Date of filing: 23.01.2024
(51) Int. Cl.: C07D 251/60, B01J 3/00

(54) **APPARATUS AND PROCESS FOR THE PRODUCTION OF MELAMINE FROM UREA**

(30) Priority: 25.01.2023 IT 202300001092
(71) Applicant: Proman AG, 8832 Wollerau Schwyz (CH)
(72) Inventor: DE AMICIS, Alberto, I-20097 San Donato Milanese (MI) (IT); DI RUOCCO, Giuseppe, I-23900 Lecco (LC) (IT); SANTUCCI, Roberto, I-21050 Gorla Maggiore (VA) (IT)
(74) Representative: De Gregori, Antonella

(57) **Abstract**

The present invention relates to an apparatus for the production of melamine from urea at high pressure and the relative process.

## Description

The present invention relates to an apparatus for the production of melamine from urea and the relative process.

The process for producing melamine from urea can be carried out either at low pressure, in the presence of catalysts, or at high pressure without the use of catalysts. In both cases the reaction is carried out at a temperature of 360-420°C and is highly endothermic. The reaction heat, negative, is approximately 93,000 Kcalories per Kmole of melamine produced.

Regardless of the reaction pressure, the transformation of urea into melamine takes place according to the following overall reaction scheme:

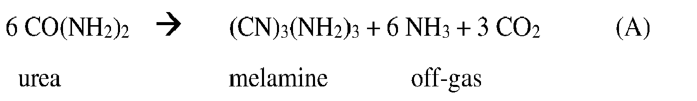

With respect to the process for producing melamine from urea at high pressure, it is believed that the most probable kinetic sequence of the intermediate reactions leading to the final formation of melamine is the following:

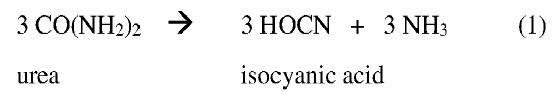

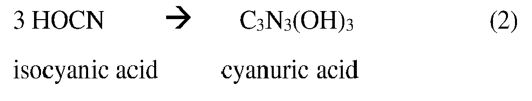

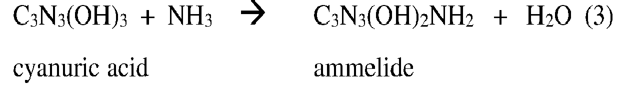

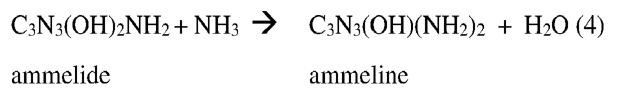

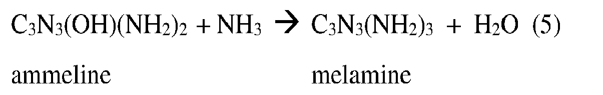

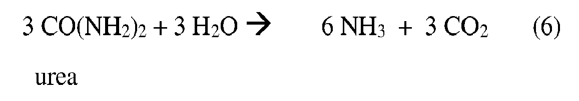

By adding the reactions from (1) to (6) the overall reaction (A) is obtained.

In the final reaction product, in addition to unreacted urea (in the case of a conversion lower than 100%), some of these intermediate products may be present, in particular ammeline and ammelide, briefly indicated hereunder by the acronym OATs (OxyAminoTriazines).

Furthermore, under the reaction conditions, the melamine produced reacts with itself with the elimination of ammonia, becoming transformed into high-molecular-weight condensation products (polycondensates) which pollute the melamine itself, lowering its purity and reducing the overall yield of the process..

The polycondensate species present in the highest quantities in the reaction product are melam and melem. They are formed according to the following overall reaction schemes:

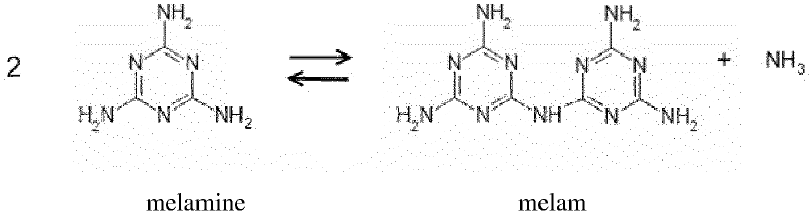

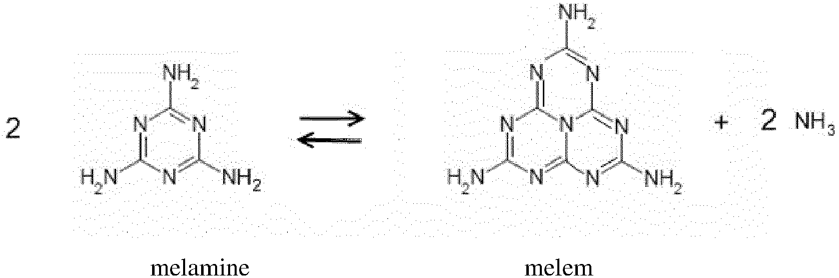

The reactions that lead to the formation of polycondensates are reversible, they take place in the liquid phase and are favoured by the low partial pressure of ammonia and by the prolonged permanence of the melamine at the temperatures in which said melamine is liquid (> 354°C). Polycondensates are produced in modest quantities under the melamine synthesis conditions, but still significant in consideration of the high degree of purity of the final product required.

In the high-pressure process, urea in the molten state, at temperatures ranging from 140 to 150°C, is introduced into a synthesis reactor, usually together with gaseous NH₃, maintained at a temperature ranging from 360 to 420°C using suitable heating means. The raw melamine leaving the reactor is then subjected to a purification treatment, typically through dissolution in water and subsequent recrystallization. In this treatment, the gaseous reaction co-products (ammonia and carbon dioxide) are recovered and the reaction by-products (essentially OATs and polycondensates) in addition to the unreacted urea are eliminated and/or separated.

In most industrial processes still currently operating, the melamine synthesis reaction is carried out at high pressure in continuous and in the liquid phase, generally in a single homogeneous reactor, normally consisting of a cylindrical container in which the reacting mass is kept under vigorous stirring thanks to the gases that develop during the reaction itself. The (negative) reaction heat is transferred to the reacting mass via appropriate heat-exchange tubes, positioned inside the reactor, within which a heat-carrier fluid circulates at a temperature higher than the reaction temperature.

Thanks to the intense state of stirring caused by the formation of the gaseous reaction by-products, the concentration of all the chemical species inside the reactor tends to acquire a practically equal value at every point of the liquid mass and the urea which is fed in continuous is rapidly mixed in the circulating reacting mass. The reaction product that is continuously extracted will therefore have a concentration identical to that of the reacting mass circulating in the reactor. Given the kinetic characteristics of the reactions involved in a system such as that described above, it may be necessary to use considerable reaction volumes which make the operation extremely expensive also because, as the reactor has to resist the highly corrosive action of the reagents and products obtained under considerably severe temperature and pressure conditions, the materials constituting this reactor and their processing are extremely expensive.

As is known to skilled persons in the field, in a single continuous reactor, perfectly mixed and characterized by homogeneous concentrations of the chemical species at every point of the reacting mass, the residence time of any portion of liquid circulating inside it varies from zero to infinite according to a distribution curve which has its maximum very close to the average residence time, the latter being defined by the ratio between the volume of the system and the feed flow-rate (urea in this case).

Under these conditions, the shorter the average residence time, the greater will be the portions of reacting mass that can exit from the reactor before the urea has been completely transformed into melamine, i.e. the smaller the reaction volume in relation to the feed flow-rate of the incoming urea.

In addition to this is the fact that, due to the practical difficulty of obtaining a state of perfect mixing, "by-pass" phenomena may occur, consisting in the leakage of unreacted urea from the reactor even before it has been completely incorporated into the circulating mass.

In the known art, a first solution proposed for overcoming the above-mentioned drawbacks consists in providing a reaction section of the plant for the production of melamine from urea which comprises a reactor, in which most of the urea is transformed into melamine, and a post reactor (also called finisher or stripper with ammonia) in which the transformation of urea into melamine is completed.
Reaction (1)

3 CO(NH₂)₂ → 3 HOCN + 3 NH₃ (1)

urea isocyanic acid is favoured by a low partial pressure of NH₃; whereas the reactions (3)-(5)

C₃N₃(OH)₃ + NH₃ → C₃N₃(OH)₂NH₂ + H₂O (3)

cyanuric acid ammelide

C₃N₃(OH)₂NH₂+ NH₃ → C₃N₃(OH)(NH₂)₂ + H₂O (4)

ammelide ammeline

C₃N₃(OH)(NH₂)₂ + NH₃ → C₃N₃(NH₂)₃ + H₂O (5)

ammeline melamine are favoured by a high partial pressure of NH₃. It is therefore important to reduce the partial pressure of CO₂ as much as possible in order to increase that of NH₃ to have maximum efficiency in the post reactor.

Consequently, in order for the transformation of urea into melamine to take place completely, the CO₂ present both in the gas phase and dissolved in the liquid phase, consisting of raw melamine, must be eliminated as much as possible in order to minimize the partial pressure of the CO₂ in the system.

In a reaction section comprising reactor and post-reactor, the separation of the reaction gases from the liquid phase normally takes place in the upper part of the reactor where the gas phase is discharged under pressure control, whereas the liquid phase enters the post-reactor, which operates at the same pressure as the reactor and which is connected to the reactor via appropriate piping, through communicating vessels.

In order to prevent the gases containing a high concentration of CO₂, present in the upper part of the reactor, from entering the post-reactor together with the liquid phase, the connection pipe between the reactor and the post-reactor has at least one end immersed in the liquid phase, thus creating a hydraulic seal, better known as hydraulic guard.

This conformation of the reaction section of the plant, however, even in its simplest implementation, has the drawback that, as the liquid phase is kept under strong stirring by the reaction gases that develop in the reactor, the raw melamine that passes from the reactor to the post reactor, in addition to the dissolved gases, incorporates and carries with it into the post reactor a considerable quantity of gas, and therefore above all a considerable quantity of CO₂. EP3838398 describes an example of this solution in which the liquid phase containing melamine, which passes from the reactor to the post reactor, substantially contains all the CO₂ (incorporated and dissolved) and therefore, in the post reactor, the stripping phase for removing the CO₂ is considerably lengthy, decreasing the efficiency of the post reactor.

In order to overcome this drawback, some technologies described in the state of the art also provide for the use of a reaction gas separator in the reaction section of the plant, positioned between the reactor and the post reactor, and connected to them via a system of pipes and valves, in which the liquid phase coming from the reactor remains in a quiet condition in the separator before reaching the post reactor, thus favouring the release of the gases incorporated therein.

This configuration of the reaction section of the plant, although allowing the release of the gases incorporated in the liquid melamine coming from the reactor, has the negative aspect in that the gas phase overlying the liquid phase inside the gas separator is rich in CO₂ which interferes negatively with the reduction of CO₂ that remains dissolved in the liquid phase, effectively reducing the benefit linked to the presence and use of the gas separator. A further negative aspect of this configuration of the reaction section of the plant, which comprises a reactor/separator/post-reactor, is the need for having three distinct pieces of equipment operating at high pressure and high temperature, connected to each other by a system of pipes and valves also operating under the same conditions, with the need for using special materials that must resist the severe operating conditions and resulting in high costs of the system.

In order to overcome the high costs that the configuration of the reaction section of the plant described above entails, reaction sections have been designed that involve combining the reactor and the post-reactor in a single device (ITMI20081776). In this solution, however, the gas phase of the post reactor is in common with the gas phase of the reactor, and is therefore rich in CO₂, and/or the raw melamine enters the post reactor still rich in dissolved and/or incorporated gases entraining with it an excessive quantity of CO₂ and thus jeopardizing the efficiency of the post reactor.

The objective of the present invention is to overcome the drawbacks indicated by the state of the art and, in particular, the objective of the present invention is to minimize the quantity of CO₂ incorporated and/or dissolved in the raw liquid melamine that is fed to the post reactor, thus increasing its efficiency.

The present invention therefore first relates to a reaction section of a plant for the high-pressure synthesis of melamine from urea which comprises an apparatus 1, having at least a perimeter wall 25, a lower wall 32' and an upper wall 32, said apparatus 1 comprising three sections: a first section A, called reaction zone or reactor, a second section B, called separation zone or gas separator and a third section C, called finishing zone or post reactor, through which a liquid stream of raw melamine flows continuously, which is produced in section A, moves from section A to section B and then from section B to section C through communicating vessels; said apparatus 1 being characterized in that
- section A or the reactor comprises a tank 2 comprising a first vertical cylindrical wall 4, said tank being suitable for allowing the high-pressure synthesis of melamine from urea;
- section C or the post-reactor comprises a tank 3 arranged concentrically with respect to section A or the reactor and comprising a second vertical cylindrical wall 5 and a lower wall 22, said tank 3 forming a volume between said section A and said perimeter wall 25, said volume being suitable for guaranteeing a stationary time of the liquid stream of raw melamine which allows stripping of the CO₂ and the completion of the reactions with the formation of purified melamine, said first wall 4 and said second wall 5 coinciding and constituting the two surfaces of the same vertical cylindrical element which separates section A from section C;
- section B of the apparatus, or separation zone, comprises a tank 9 comprising a third wall 6 and a fourth wall 7 which define an annular volume between section A and section C, said tank 9 being supported by the upper portion 8 of the vertical cylindrical element consisting of the first wall 4 and second wall 5,

wherein said third wall 6 coincides with an upper portion of the first wall 4 and said fourth wall 7 coincides with an upper portion of the second wall 5;
wherein said section B and said section C further comprise a separation septum 10 comprising:
   - a circular crown 20 connected to the perimeter wall 25 of the apparatus in correspondence with section C of the apparatus 1, wherein said circular crown 20 is suitable for defining and delimiting an upper portion or head section 18 of said third section C, and
   - a vertical cylindrical wall 13 extending, starting from the end of said circular crown 20, in correspondence with section B and suitable for defining in the second section B two zones B' and B", respectively called first calm zone B' and second calm zone B", said first calm zone B' being adjacent to the first reaction section A, whereas said second calm zone B" is adjacent to the third finishing section C, said first calm zone B' being suitable for receiving from the reaction zone A, the liquid phase composed of liquid melamine containing incorporated CO₂, dissolved CO₂ and intermediate reaction products and separating most of the CO₂ incorporated in the liquid phase and said second calm zone B" being suitable for separating the remaining incorporated CO₂ and feeding said liquid solution of raw melamine, almost completely free of incorporated CO₂, still containing dissolved CO₂ and intermediate reaction products, to the finishing section or post reactor C;
said apparatus 1 being further characterized in that the upper portion or head section 11 of the tank 2 of section A comprises outlet means 12 for releasing the gaseous phases coming from section A and section B' and the upper portion or head section 18 of the tank 3 of section C comprises outlet means 14 for releasing the gaseous phases coming from section C and section B", said upper portions or head spaces 11, 18 of tanks 2, 3 of sections A and C not communicating directly with each other.

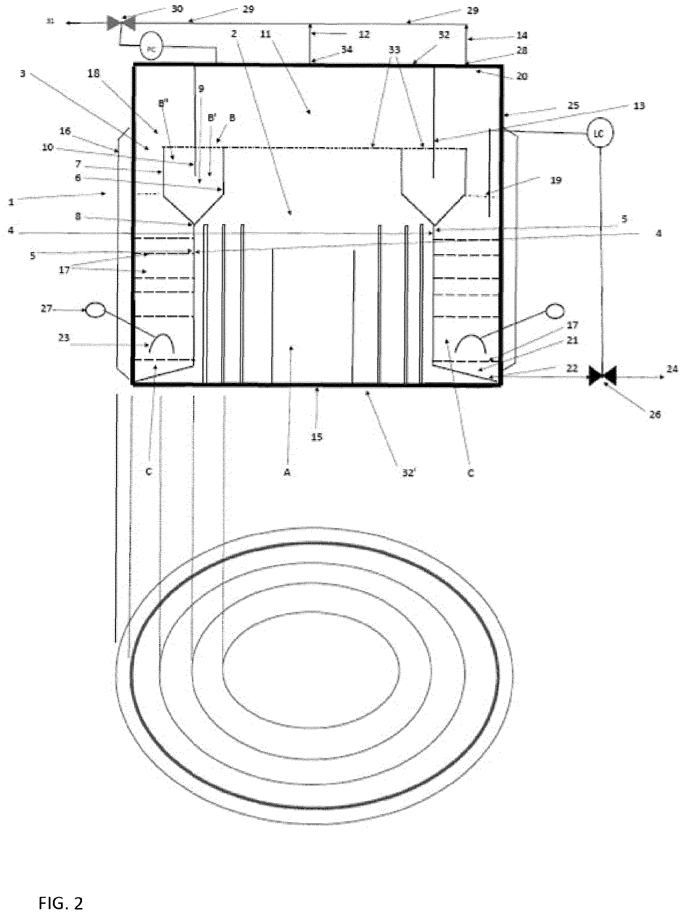

In the reaction section according to the present invention, a portion of the upper wall 32 of the apparatus 1 can coincide with said circular crown 20. This solution is represented by the embodiment of figure 2.

In the present description, the definition "most of the CO₂ incorporated in the liquid phase" means that at least 90% of the incorporated CO₂ is separated, whereas the definition "the remaining CO₂ incorporated" means that the remaining approximately 8.5-9.3% of the incorporated CO₂ is separated. Finally, the definition "liquid solution of raw melamine, almost completely free of incorporated CO₂" refers to a liquid solution of melamine containing a maximum of approximately 0.7-1.5% of incorporated CO₂.

Said vertical cylindrical wall 13 preferably extends between said circular crown 20 and said walls 6 and 7 of section B.

Upper portion or headspace 11 of the tank 2 of section A refers to the space defined below by the level 33 of the liquid solution of raw melamine in the tank 2 and above by the upper wall 32 of the apparatus 1.

Upper portion or headspace 18 of the tank 3 of section C refers to the space defined below by the level 19 of the liquid solution of raw melamine in the tank 3 and above by the circular crown 20, which therefore constitutes the upper wall that closes the tank 3 of section C, by the portion of vertical wall 13 not immersed in the liquid solution of raw melamine, and by the portion of wall 25 of the apparatus 1 not immersed in the liquid solution of raw melamine and which therefore exits from the level 19.

Said perimeter part 25 of the apparatus 1 according to the present invention is preferably cylindrical.

Said tank 3 preferably forms an annular volume between said section A and said perimeter wall 25.

Said vertical cylindrical wall 13 preferably extends starting from the end of said circular crown 20, in correspondence with section B, at least partially inside the annular volume defined by the tank 9 and more preferably below the level of the liquid solution of raw melamine 33 in tank 9 of section B.

Said circular crown 20, connected to the perimeter wall 25 of the apparatus 1 in correspondence with section C of the apparatus 1, is suitable for defining an upper portion or head section 18 of said third section C and of section B" of the apparatus 1.

The two calm zones B' and B" in section B are respectively arranged in a position adjacent to section A and in a position adjacent to section C.

Said circular crown 20 preferably delimits an upper portion or head section 18 of the third section C and section B".

The separation septum 10 is therefore a cylindrical septum which defines and delimits the two calm zones B' and B" in section B, said calm zones B' and B" being of equal dimensions or different dimensions, the calm zone B" preferably being smaller in size than the calm zone B', the septum 10 being positioned close to the fourth wall 7 of section B.

The septum 10, is preferably, immersed in the tank 9 of section B or gas separator, and guarantees that the separation of the gas phase of the reactor from the gas phase of the post reactor is maintained.

The septum 10 is positioned so that the upper surface of the liquid phase of the calm zone B" has a size that varies from about 1/3 to about 1/10, it is preferably equal to about 1/8 compared to the upper surface of the liquid phase of the calm section B'.

In one embodiment, said tank 9 of the second separation section B or gas separator has a cylindrical shape with a preferably conical base, whose vertex is supported by the upper end 8 of the wall 5 of the tank 3 of section C and of the wall 4 of the tank 2 of section A, wall 4 and wall 5 coinciding and constituting a vertical cylindrical element.

The solution according to the present invention is particularly advantageous as it allows the gas phases to be kept separate inside the apparatus and specifically between the reaction section A or reactor and the finishing section C or post reactor, also feeding a liquid raw melamine into the latter wherein the quantity of CO₂ incorporated and/or dissolved is minimal.

The present invention also relates to a process for the production of melamine from urea at high pressure, wherein a liquid stream of raw melamine, emerging from a first synthesis step of melamine from urea in a first reaction section A, flows in continuous into at least two successive sections B and C communicating with each other and positioned in sequence with respect to the flow of the liquid stream of raw melamine, wherein
in the separation section B, the separation of the gas phase incorporated in the liquid phase of raw melamine is effected by remaining stationary in two successive calm zones B' and B", in the first calm zone B', adjacent to the reaction section A and in contact with the gaseous phase of the reaction section A, separating most of the gas incorporated in the liquid phase and in the second calm zone B", adjacent to section C and in contact with the gaseous phase of section C, separating the remaining incorporated gas;
in the finishing section or post-reactor C, the finishing of the transformation from urea into melamine is effected and the separation of the gas phase dissolved in the liquid phase of raw melamine is carried out by feeding at least one gaseous stream of NH₃ and the separation of the residual gas phase still incorporated in the liquid phase is completed.

The present invention is described hereunder with reference to the following schematic figures which are illustrative and non-limiting of the protection scope defined by the enclosed claims:
figure 1: schematic representation of an apparatus according to a first embodiment of the present invention;
figure 2: schematic representation of an apparatus according to a second embodiment of the present invention;

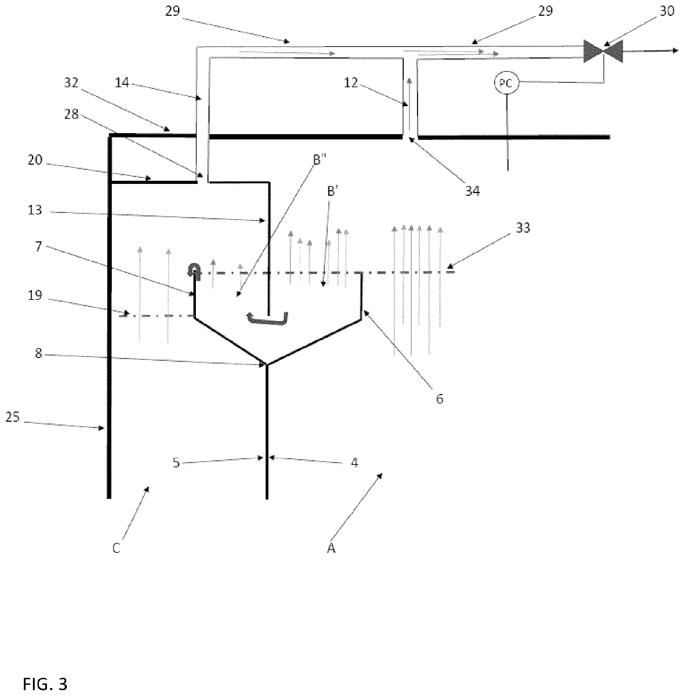

figure 3: schematic representation of a detail of the apparatus according to the first embodiment represented in figure 1.

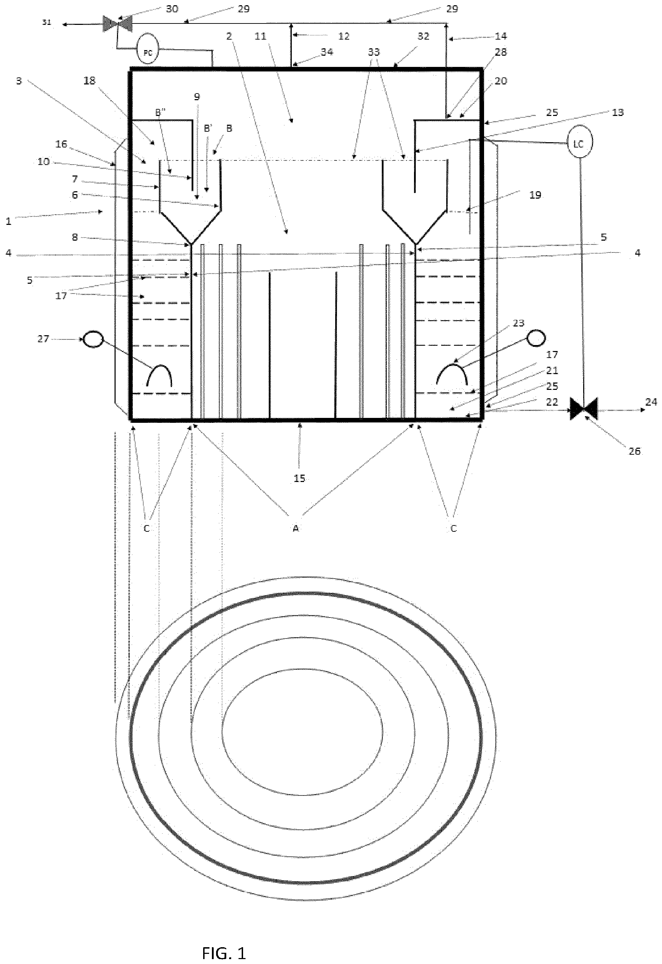

In accordance with the embodiment represented in figure 1, in the tank 2 of the first section A of the apparatus 1 according to the present invention, also called reaction zone or reactor, the urea fed from below through inlet means 15 is transformed into melamine by the well-known overall reaction

6 CO(NH₂)₂ → (CN)₃(NH₂)₃ + 6 NH₃ + 3 CO₂ (A)

urea melamine off-gas and the large quantity of gas that develops from this reaction is evident. The reaction shows in fact that for each molecule of melamine, six molecules of NH₃ and three molecules of CO₂ are formed which, developing within the liquid mass, keep said mass under very strong stirring, consequently preventing an adequate separation of the gases from the liquid phase.

In the tank 2 of the first section A of the apparatus 1 according to the present invention, the gaseous phase (first gaseous phase) which is located above the liquid phase, is composed of the reaction gases and is therefore rich in CO₂ which, contributing with its partial pressure to the total pressure of the reactor, maintains, in fact, a significant quantity of CO₂ dissolved in the liquid phase which adds to the quantity of CO₂ that remains incorporated in said liquid phase.

As mentioned previously, in order to have a complete transformation of urea into melamine, it is necessary for the quantity of CO₂ in the liquid phase to be reduced as much as possible: both forms of CO₂ present in the liquid phase and specifically both the CO₂ incorporated and the CO₂ dissolved in the liquid phase of raw melamine must therefore be removed and eliminated.

The apparatus 1 according to the present invention therefore advantageously makes it conveniently possible to obtain the separation first of the CO₂ incorporated and then of the dissolved CO₂. This separation takes place in the second and third sections of the apparatus according to the present invention and specifically in section B called separation zone or gas separator and in section C called finishing zone or post reactor.

In order to optimize the functioning of the second section B of the apparatus according to the present invention, i.e. the separation zone or gas separator, it should be considered that the composition of the gas incorporated in the liquid phase is the same composition as the gaseous phase that has been separated from the liquid phase in the upper portion of the reaction zone or reactor, it is therefore rich in CO₂ and it is therefore essential that the passage to the third section C, called the finishing zone or post-reactor, of the gas incorporated in the liquid phase coming from section A and which is released in the second section B of the apparatus, be prevented or reduced to a minimum.

By reducing to a minimum or preventing this gas rich in CO₂ from passing from the second section B to the third section C of the apparatus, it is in fact possible to reduce its presence in the gaseous phase that is formed in the upper portion of the third section C of the apparatus or finishing zone, consequently reducing the partial pressure of the CO₂ and thus facilitating the removal of the dissolved CO₂, by stripping with NH₃ in the third section of the apparatus or post reactor.

In order to achieve the above objective, the second section B of the apparatus, or separation zone, is characterized by the presence in the upper portion of the tank 9 of the section itself of two different zones B' and B", respectively called the first calm zone B' and the second calm zone B", defined and separated by a separation septum 10.

This septum 10, composed of the circular crown 20 and the vertical wall 13, defines in the upper portion of the tank 9 of the second section B, two distinct zones B' and B", wherein section B' is adjacent to the first reaction section A, whereas section B" is adjacent to the third finishing section C.

The first calm zone B' receives from the reaction zone A, the liquid phase composed of liquid melamine containing incorporated CO₂, dissolved CO₂ (in a quantity that depends on the partial pressure of the CO₂ in the gaseous phase of section A) and intermediate reaction products and separates most of the gases incorporated in the liquid phase, containing CO₂: this is made possible by the fact that the first calm zone B' is characterized by the lack of turbulent circulation of the liquid phase whereas, with respect to the quantity of dissolved gases, as the calm zone B' is arranged in a position adjacent to section A, and as the gaseous phase of the calm zone B' is in contact with and therefore common to the gaseous phase of section A, consisting of the reaction gases rich in CO₂, the quantity of gases dissolved in the liquid phase of the calm zone B' does not undergo variations with respect to the quantity contained in the liquid phase of section A as they are subject to the same partial pressures in the gaseous phase.

In the second calm zone B", however, the gases containing CO₂, still incorporated in the liquid phase, are separated, the quantity of gases dissolved in the liquid phase is reduced and the gases released from the liquid phase and the liquid solution of raw melamine, almost completely free of incorporated CO₂, but still containing dissolved CO₂ and intermediate reaction products, are fed to the finishing or post-reactor section C, where the dissolved CO₂ is also removed by stripping with NH₃ and the transformation reaction of the residual urea into melamine is completed; this is made possible by the fact that the second calm zone B" is arranged in a position adjacent to section C, therefore in contact with the gaseous phase coming from section C consisting of a stripping stream of NH₃, poor in CO₂ and therefore having a reduced partial pressure of CO₂, which facilitates the removal of dissolved CO₂ and the completion of the transformation reaction of urea into melamine.

This solution is therefore particularly advantageous as it allows there to be in the finishing section C, a gaseous phase having a very reduced CO₂ content and enables a liquid solution of raw melamine, almost completely free of incorporated CO₂, still containing a reduced quantity of dissolved CO₂ and intermediate reaction products, to be fed to section C, with a consequent increase in the efficiency of the finishing section C: the stripping phase in the post reactor is in fact reduced and all the remaining volume of the post reactor can be exploited for completing the transformation reaction of urea into melamine with an increase in the efficiency of the melamine synthesis process.

The lower portion of the tank 9 of the second section B is single and does not provide distinctions between the area adjacent to the reaction section A and the area adjacent to the finishing section C.

The particular conformation of section B is suitable for preventing or in any case reducing to a minimum the passage of gases from zone B' to zone B", whereas the passage of the liquid phase is not limited in any way. The liquid phase consisting of raw melamine is fed through communicating vessels from the first section A of the apparatus or reactor to the first zone B' of the second section B of the apparatus (gas separator) and this liquid phase then flows, again through communicating vessels, from the first zone B' to the second zone B" of the second section B of the apparatus; as the whole system is in equipressure and the side walls 6, 7 of the tank 9 of section B reach the same height, the level of the liquid phase in the reaction section A, in the first calm zone B' and in the second calm zone B" of the second section B is at the same height.

The second section B of the apparatus, i.e. the separation section or gas separator, therefore comprises two different stationary zones in the upper portion of the tank 9: the first calm zone B' which receives the liquid phase from the reaction zone A or reactor composed of liquid melamine containing incorporated CO₂, dissolved CO₂ and intermediate reaction products and separates most of the gas incorporated in the liquid phase. This gas, freed from the liquid phase, joins the gaseous phase coming from the first section A composed of the reaction gases. The liquid phase of melamine, purified from most of the incorporated gas, then flows towards the second calm zone B" of the second section B, where the remaining incorporated gas is separated and joins the gaseous phase which is formed in the third section C or post reactor. The raw melamine, almost completely free of incorporated gas, still containing dissolved CO₂ and intermediate reaction products, enters, due to overflow, the tank 3 of the third section C of the apparatus 1 or post reactor.

The gaseous phase that is formed in the third section C and to which said residual incorporated gas which is separated in the second calm zone B" is joined, is composed of stripping NH₃ which, fed into the lower portion of the tank 3 of the third section C, rises through said tank 3, encountering the melamine which, flowing due to overflow from the second calm zone B" of the tank 9 of the second section B, enters the upper portion of the tank 3 of the third section C and descends towards the lower portion of the tank 3 of the third section C, thus encountering in countercurrent the stripping NH₃ rising from below. The stripping NH₃ removes the CO₂ dissolved in the liquid stream of raw melamine and carries it towards the upper portion of the tank 3 of the third section C or post reactor, thus forming the gaseous phase of the post reactor.

The first calm zone B' receives from the reaction zone A, the liquid phase composed of liquid melamine containing incorporated CO₂, dissolved CO₂ (in a quantity that depends on the partial pressure of the CO₂ in the gaseous phase of section A) and intermediate reaction products and separates most of the gases incorporated in the liquid phase, containing CO₂: this is made possible by the fact that the first calm zone B' is characterized by the lack of turbulent circulation of the liquid phase whereas, with respect to the quantity of dissolved gases, as the calm zone B' is arranged in a position adjacent to section A, and as the gaseous phase of the calm zone B' is in contact with and therefore common to the gaseous phase of section A, consisting of the reaction gases rich in CO₂, the quantity of gases dissolved in the liquid phase of the calm zone B' does not undergo variations with respect to the quantity contained in the liquid phase of section A as they are subject to the same partial pressures in the gaseous phase.

In the second calm zone B", however, the gases containing CO₂, still incorporated in the liquid phase, are separated, the quantity of gases dissolved in the liquid phase is reduced and the gases released from the liquid phase and the liquid solution of raw melamine, almost completely free of incorporated CO₂, but still containing dissolved CO₂ and intermediate reaction products, are fed to the finishing section or post-reactor C, where the dissolved CO₂ is also removed by stripping with NH₃ and the transformation reaction of the residual urea into melamine is completed; this is made possible by the fact that the second calm zone B" is arranged in a position adjacent to section C, therefore in contact with the gaseous phase coming from section C consisting of a stripping stream of NH₃, poor in CO₂ and therefore having a reduced partial pressure of CO₂, which facilitates the removal of dissolved CO₂ and the completion of the transformation reaction of urea into melamine.

Section B or gas separator can also provide drainage holes in the lower portion of the side wall 7 which divides it from section C or post-reactor, said drainage holes allowing the tank 9 of section B to be emptied during stoppages of the apparatus 1. These holes, as already indicated, are arranged on the side of section C or post reactor, in order to avoid the possible entry of reaction gases from the reactor section A to section B or gas separator.

Section C or post-reactor is composed of a tank 3 with a vertical cylindrical seal which is positioned externally to the tube bundle of the reactor A and fixed to the lower portion of the perimeter wall 25, guaranteeing a seal so as to prevent the liquid phase of the reactor A from entering the post reactor C in the lower portion or tail section of the same. Said section C forms a substantially annular volume around the tank 2 of section A, so as to guarantee a stationary time suitable for obtaining the stripping of the CO₂ and the completion of the reactions with the formation of purified melamine which is extracted from the bottom of the post reactor by a pipe with a level control valve.

More specifically, section C or post reactor is composed of
- a vertical cylindrical sealed tank 3;
- a heating jacket 16, preferably with molten salts, arranged externally with respect to said vertical cylindrical tank;
- a plurality of plates 17 provided with pass-through holes, said plates 17 being arranged one above the other in a configuration reciprocally spaced vertically to delimit a plurality of sections inside the sealed tank, each section communicating with the adjacent section through said pass-through holes;
- a head section 18 of the sealed tank 3 of the post-reactor, said head section 18 being defined between the level of the raw melamine liquid phase 19 and the upper wall of the sealed tank 3 of the post-reactor C, coinciding with the circular crown 20, possibly coinciding with the corresponding portion of the upper wall 32 of the apparatus 1, and by the cylindrical side wall portion 25 between the level of the liquid phase and the upper wall;
- a tail section 21 of the sealed tank 3, said tail section 21 being defined between the lower plate 17 of said plurality of plates and a lower wall 22 of the tank 3 and by the cylindrical side wall portion 25 included between the lower plate 17 of said plurality of plates and the lower wall 22;
- one or more intermediate sections between the head section 18 and the tail section 21 defining a finishing region;
- the intermediate section adjacent to the head section 18 and at least the section adjacent to it, defining a CO₂ stripping region;
- inlet means 23 of a gaseous stream of NH₃ inside the tank 3 of said post-reactor, arranged in any section below the head section 18, preferably in the section adjacent to the tail section 21 of the tank 3;
- outlet means 24 of the purified melamine from inside the tank 3 of said post-reactor C, arranged in the tail section 21 of the tank 3.

The term "a plurality of plates" refers to at least two plates, whereas the term "a plurality of sections" refers to at least three sections, as the number of sections defined by the plates is always one unit higher than the number of plates.

Tail section 21 of the post-reactor tank 3 refers to the section of the sealed tank 3 of the post-reactor C, defined by the lower plate 17 of the plurality of plates, the lower wall 22 of the sealed tank of the post-reactor C and the portion of cylindrical side wall 25 included between said lower plate and lower wall 22, therefore arranged below the lower plate 17 provided with pass-through holes.

The plates 17 therefore define, in the sealed tank 3 of the post-reactor C, a series of successive sections through which the liquid stream flows downwards and the gaseous stream of NH₃ flows upwards.

The gas separator B and the post-reactor C are positioned in the order indicated downstream of a reactor A in which a first reaction step of a melamine production process from urea is carried out, said first step being a pyrolysis reaction of urea, generally, effected according to processes known in the state of the art in a single shaft reactor, as described, for example, in US'294 and US'074.

The term "downstream" with reference to the position of the reactor, separator and post-reactor indicates the relative position with respect to the movement direction of the liquid phase or stream of raw melamine which then moves from the reactor A where it is synthesized, to the separator B and finally from the separator B to the post-reactor C.

The liquid stream of raw melamine produced in the first step of the melamine production process from urea and which has passed through the gas separator B, as previously described, flows inside the post-reactor C.

In particular, the post-reactor C comprises outlet means 24 for the liquid melamine streams and outlet means 14 for the gaseous phase, which keeps the level of the liquid phase as well as the pressure of the gaseous phase constant.

The purified melamine is extracted from the bottom of the post reactor C via a pipe 24 with a level control valve 26.

The inlet means 27 of the gaseous stream of NH₃ inside the tank 3 of the post-reactor C preferably comprises a distributor means 23.

A heating jacket 16 may also be present, preferably with molten salts, arranged externally with respect to the vertical cylindrical sealed tank 3 of the post-reactor C, which provides inlet and outlet means for the heating fluid (not shown in the figure) and is characterized by the presence of a ΔT between the inlet temperature of the heating fluid and the outlet temperature, corresponding to the heat supplied to the post-reactor C.

As already mentioned, two or more of the intermediate sections between the head section 18 and the tail section 21 of the tank 3 of the post-reactor C define a finishing region in which the transformation reaction of the pyrolysis intermediates (essentially OATs) into melamine, with a consequent increase in selectivity, and the conversion reaction of unreacted urea into melamine, with a consequent increase in yield, take place contemporaneously.

The post-reactor can provide a number of perforated plates which varies from 3 to 40 and which defines from 4 to 41 sections in the sealed tank of the post-reactor itself, preferably the number of perforated plates varies from 4 to 20 and defines from 5 to 21 sections in the sealed tank of the post-reactor.

The apparatus 1 according to the present invention is further characterized in that the upper portion or head section 11 of the tank 2 of section A comprises outlet means 12 for the discharge of the gaseous phases coming from section A and section B' and the upper portion or head section 18 of the tank 3 of section C comprises outlet means 14 for the discharge of the gaseous phases coming from section C and section B", said upper portions or head spaces 11, 18 of the tanks 2, 3 of sections A and C not being directly communicating with each other.

The head section 18 of the tank 3 of the post reactor C comprises one or more outlet points 28 of the gaseous phase, connected to the outside of the apparataus 1 according to the present invention by means of pipes 14 which enter a further pipe 29, called connection pipe.

The gaseous phase coming from section A or reactor also reaches this connection pipe 29: more specifically, the head section 11 of the tank 2 of the reactor A comprises one or more outlet points 34 of the gaseous phase, connected to the outside of the the apparatus 1 according to the present invention by means of pipes 12 which are in turn connected to the connection pipe 29. The gaseous phases, thus combined in the connection pipe 29, are then sent, through the pressure control valve 30 of the apparatus 1, to the sections downstream of the plant 31 (not shown in the figure) for the subsequent necessary treatment.

As, in the connection pipe 29, the two gaseous phases, coming respectively from tank 2 of the reactor A and from area B' and from tank 3 of the post reactor C and from area B" are combined and controlled by a single control valve 30, the apparatus 1 according to the present invention guarantees that the two gas phases are at the same pressure.

The outlet means 12 of the gaseous phase from tank 2 of section A or reactor are connected to the connection pipe 29 downstream with respect to the position in which the outlet means 14 of the gaseous phase from tank 3 from section C are connected: this avoids a return of CO₂ into the gaseous phase coming from section C or post reactor, ensuring that the flow of the gaseous phase is directed towards the pressure control valve 30 which is the only outlet point of the system.

The term "downstream" should be considered with respect to the movement direction of the gaseous phase (fluid) in the connection pipe 29.

Figure 2 schematically represents a detail of figure 1, wherein the same elements are indicated with the same numbers as figure 1. The arrows F inside the tank 9 of the separation section B show the path of the liquid phase of raw melamine. The vertical arrows in the tanks 2, 3 and 9, of sections A, B and C respectively, in the pipes 12 and 14 and the horizontal arrows in the connection pipe 29 represent the gases moving away from the liquid phase of raw melamine and coming out of the apparatus 1 according to the present invention.

The apparatus and process according to the present invention have various advantages, in particular:
- the possibility of eliminating both the incorporated CO₂ and the dissolved CO₂ from the stream of raw melamine with a consequent increase in the selectivity of the melamine synthesis process;
- that is, the possibility of having in the finishing section C, a gaseous phase with a very low CO₂ content and feeding the liquid solution of raw melamine, almost completely free of incorporated CO₂, containing a reduced quantity of dissolved CO₂ and intermediate reaction products, with a consequent increase in the efficiency of the finishing section C: the stripping phase in the post reactor is in fact reduced and all the remaining volume of the post reactor can be exploited for completing the transformation reaction of urea into melamine with an increase in efficiency of the melamine synthesis process;
- the possibility of keeping the gas phases separate inside the apparatus and specifically between the reaction section (A) or reactor and the finishing section (C) or post reactor, also feeding into the latter a raw liquid melamine in which the quantity of incorporated and/or dissolved CO₂ is minimal;
- the possibility of reducing back-mixing phenomena in the post reactor of the conversion reaction products with a consequent increase in the transformation rate and therefore a reduction in the necessary reaction volume, with the same yield;
- the possibility of producing melamine with a high degree of purity (above 99.9%) in a single and compact apparataus with a considerable reduction in investment costs;
- much lower costs linked to a plant in which a single apparatus is envisaged comprising the three sections described, instead of a plant with three distinct pieces of equipment, together with a considerable reduction in the necessary spaces;
- in the case of limited spaces, the apparatus according to the present invention allows a reactor to be replaced with a system comprising reactor-separator-post-reactor with the obvious advantages specified above;
- the apparatus can be relatively easily integrated also into existing systems, regardless of the type of configuration adopted (revamping).

## Claims

1. A reaction section of a plant for the high-pressure synthesis of melamine from urea comprising an apparatus (1), having at least one perimeter wall (25), preferably cylindrical, a lower wall (32') and an upper wall (32), said apparatus (1) comprising three sections: a first section (A), called reaction zone or reactor, a second section (B), called separation zone or gas-separator and a third section (C), called finishing zone or post-reactor, through which a liquid stream of raw melamine that is produced in section (A) flows continuously, moving from section (A) to section (B) and then from section (B) to section (C) through communicating vessels;
said apparatus (1) being **characterized in that**
- section (A) or the reactor comprises a tank (2) comprising a first vertical cylindrical wall (4), said tank being suitable for allowing the high-pressure synthesis of melamine from urea;
- section C or the post-reactor comprises a tank (3) arranged concentrically with respect to section (A) or the reactor and comprising a second vertical cylindrical wall (5) and a lower wall (22), said tank (3) forming a volume between said section (A) and said perimeter wall (25), said volume being suitable for guaranteeing a stationary time of the liquid stream of raw melamine which allows stripping of the CO₂ and completion of the reactions with the formation of purified melamine,
said first wall (4) and said second wall (5) coinciding and constituting the two surfaces of a same vertical cylindrical element that separates section (A) from section (C);
- section (B) of the apparatus (1), or separation zone, comprises a tank (9) comprising a third wall (6) and a fourth wall (7) which define an annular volume between section (A) and section (C), said tank (9) being supported by the upper portion (8) of the vertical cylindrical element consisting of the first wall (4) and the second wall (5),
wherein said third wall (6) coincides with an upper portion of the first wall (4) and said fourth wall (7) coincides with an upper portion of the second wall (5); wherein said section (B) and said section (C) further comprise a separation septum (10) comprising:
- a circular crown (20) connected to the perimeter wall (25) of the apparatus in correspondence with section (C) of the apparatus (1), wherein said circular crown (20) is suitable for defining and delimiting an upper portion or head section (18) of said third section (C), and
- a vertical cylindrical wall (13) extending from the end of said circular crown (20), in correspondence with section (B) and suitable for defining in the second section (B) two zones (B') and (B"), respectively called first calm zone (B') and second calm zone (B";),
said first calm zone (B') being adjacent to the first reaction section (A), whereas said second calm zone (B") is adjacent to the third finishing section (C), said first calm zone (B') being suitable for receiving from the reaction zone (A), the liquid phase composed of liquid melamine containing incorporated CO₂, dissolved CO₂ and intermediate reaction products and separating most of the CO₂ incorporated in the liquid phase and said second calm zone (B") being suitable for separating the remaining incorporated CO₂ and feeding said liquid solution of raw melamine, almost completely free of incorporated CO₂, still containing dissolved CO₂ and intermediate reaction products, to the finishing section or post reactor C;
said apparatus (1) being further **characterized in that** the upper portion or head section (11) of the tank (2) of section (A) comprises outlet means (12) for releasing the gaseous phases coming from section (A) and section (B') and the upper portion or head section (18) of the tank (3) of section (C) comprises outlet means (14) for releasing the gaseous phases coming from section (C) and section (B"), said upper portions or head spaces (11), (18) of tanks (2), (3) of sections (A) and (C) not communicating directly with each otherther.

2. The reaction section according to claim 1, wherein a portion of the upper wall (32) of the apparatus (1) coincides with said circular crown (20).

3. The reaction section according to one or more of the previous claims, wherein said tank (3) forms an annular volume between said section (A) and said perimeter wall (25).

4. The reaction section according to one or more of the previous claims, wherein said vertical cylindrical wall (13) extends starting from the end of said circular crown (20), in correspondence with section (B), at least partially within the annular volume defined by the tank (9).

5. The reaction section according to one or more of the previous claims, wherein said vertical cylindrical wall (13) extends starting from the end of said circular crown (20), in correspondence with section (B), at least partially within the annular volume defined by the tank (9) below the level of the liquid solution of raw melamine (33) of section (B).

6. The reaction section according to one or more of the previous claims, wherein said circular crown (20) delimits an upper portion or head section (18) of the third section (C) and of section (B").

7. The reaction section according to one or more of the previous claims, wherein said separation septum 10 is a cylindrical septum that defines and delimits the two calm zones (B') and (B") in section (B), said calm zones (B') and (B") having the same dimensions or different dimensions, the calm zone (B") preferably being smaller in size than the calm zone (B'), the septum (10) being positioned near the fourth wall (7) of section (B).

8. The reaction section according to one or more of the previous claims, wherein
the septum (10) is positioned so that the upper surface of the liquid phase of the calm zone (B") has a size ranging from about 1/3 to about 1/10, and is preferably equal to about 1/8 with respect to the upper surface of the liquid phase of the calm section (B').

9. The reaction section according to one or more of the previous claims, wherein the outlet means (12) for the discharge of the gaseous phases coming from section (A) and from section (B') and the outlet means (14) for the discharge of the gaseous phases coming from section (C) and from section (B") are pipes that are introduced into a further pipe 29, called connection pipe.

10. The reaction section according to one or more of the previous claims, wherein
the outlet means (12) of the gaseous phases coming from section (A) and from section (B') are connected to the connection pipe (29) downstream of the position in which the outlet means (14) of the gaseous phases coming from section (C) and from section (B") are connected, downstream with respect to the movement direction of the flow of the gaseous phases in the pipe (29).

11. The reaction section according to one or more of the previous claims, wherein the connection pipe (29) is controlled by a single control valve (30), suitable for ensuring that the gaseous phases fed through the outlet means (12) and through the outlet means (14) are at the same pressure.

12. A process for the high-pressure production of melamine from urea, wherein a liquid stream of raw melamine, exiting from a first synthesis step of melamine from urea in a first reaction section (A), flows continuously in at least two successive sections (B) and (C) communicating with each other and positioned in sequence with respect to the flow of the liquid stream of raw melamine, wherein
in the separation section (B), the separation of the gaseous phase or CO₂ incorporated in the liquid phase of raw melamine is carried out by remaining stationary in two successive calm zones (B') and (B"), in the first calm zone (B'), adjacent to the reaction section (A), in contact with the gaseous phase of the reaction section (A), separating most of the CO₂ incorporated in the liquid phase and in the second calm zone ( B"), adjacent to section (C), in contact with the gaseous phase of the reaction section (C), separating the remaining incorporated CO₂;
in the finishing section or post-reactor (C) the finishing of the transformation from urea to melamine is effected and the separation of the dissolved gaseous phase in the liquid phase of raw melamine is carried out by feeding at least one gaseous stream of NH₃, and the separation of the residual gaseous phase still incorporated in the liquid phase is completed.
